# EUROPEAN PATENT APPLICATION

(11) **EP 2 514 459 A1**
(43) Date of publication of application: **24.10.2012**
(21) Application number: 11163406.9
(22) Date of filing: 21.04.2011
(51) Int. Cl.: A61M 5/315, A61M 5/00

(54) **Medicated module for a drug delivery device**

(71) Applicant: Sanofi-Aventis Deutschland GmbH, 65929 Frankfurt (DE)
(72) Inventor: Young, Alasdair George, OX7 5ER, Chipping Norton, Oxfordshire (GB); Senior, James, Warwick, Warwickshire, CV34 4JZ (GB); De Sausmarez Lintell, Daniel Thomas, Rugby, Warwickshire, CV23 9EQ (GB); Mercer, David Richard, Warwickshire, CV31 3BY (GB)

(57) **Abstract**

A medicated module including a slidable bung that comprises a bypass flow path and a medicament cavity. The medicament cavity stores a second medicament. The medicated module also includes a bung housing for housing the slidable bung, and the bung housing comprises an inlet and an outlet. The slidable bung is configured to slide through the housing from a priming state to a dispensing state. In the priming state, the bypass flow path is in fluid communication with the inlet and outlet. Further, in the dispensing state, the medicament cavity is in fluid communication with the inlet and the outlet.

## Description

### Field of the Present Patent Application

This present patent application relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Specifically, this application concerns a medicated module that may be used with a drug delivery device, where the medicated module includes a slidable bung.

### Background

Certain disease states require treatment using one or more different medicaments. Some drug compounds need to be delivered in a specific relationship with each other in order to deliver the optimum therapeutic dose. Applicants' proposed concept is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two medicaments or active agents simultaneously. The two active agents may interact with each other during the long-term, shelf life storage of the formulation. Therefore, it is advantageous to store the active components separately and only combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more actives may require a titration period to gradually introduce a patient up to a "maintenance" dose. A further example would be if one active requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more than one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments.

Accordingly, there exists a strong need to provide devices and methods for the delivery of two or more medicaments in a single injection or delivery step that is simple for the user to perform. Applicants' proposed concept overcomes the above-mentioned problems by providing separate storage containers for two or more active drug agents that are then only combined and/or delivered to the patient during a single delivery procedure. Setting a dose of one medicament automatically fixes or determines the dose of the second medicament (i.e. non-user settable). Applicants' proposed concept also gives the opportunity for varying the quantity of one or both medicaments. For example, one fluid quantity can be varied by changing the properties of the injection device (e.g. dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime.

Additionally, in some cases, accurate filling of a medicament reservoir for a medicated module may be difficult. For instance, accurate filling of the medicament and the incorporation of an amount of additional 'head-space' in the cavity in order to accommodate manufacturing and assembly tolerances may increase manufacturing costs. Further, it may be difficult to prevent air from entering the medicament cavity of the medicated module. There is therefore also a need for a medicated module configured to prevent air from entering the medicament cavity of the medicated module.

These and other advantages will become evident from the following more detailed description of the invention.

### SUMMARY

The presently proposed devices and methods allow for complex combinations of multiple drug compounds within a single drug delivery system. Further, the presently proposed devices and methods allow the user to set and dispense at least two medicaments through one single dose setting mechanism and a single dispense interface. This single dose setter controls the dose setting mechanism of the device such that a predefined combination of the individual medicaments is delivered when a single dose of one of the medicaments is set and dispensed through the single dispense interface.

By defining the therapeutic relationship between the individual medicaments, the proposed delivery device and delivery methods help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or gases or powders that are capable of flowing and that change shape at a steady rate when acted upon by a force tending to change its shape. Alternatively, one or both of the medicaments may be a solid that is carried, solubilized or otherwise dispensed with another fluid medicament.

Applicants' proposed concept is of particular benefit to users with dexterity or computational difficulties as the single input and associated predefined therapeutic profile removes the need for them to calculate their prescribed dose every time they use the device and the single input allows considerably easier setting and dispensing of the combined compounds.

In an example, a master drug compound, such as insulin, contained within a multiple dose, user selectable drug delivery device could be used with a single use, user replaceable, medicated module that contains a single dose of a secondary medicament and the single dispense interface. When connected to the primary drug delivery device, the secondary compound is activated/delivered on dispense of the primary compound. Although the present application specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with Applicants' proposed method and system.

For the purposes of Applicants' proposed method and system the term "insulin" shall mean insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂), Exendin-3, Liraglutide, or AVE001 0 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH₂).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In accordance with an embodiment of Applicants' disclosure, a medicated module is configured to minimize or eliminate the presence of air in the primary medicament cavity in a reservoir of the medicated module. Similarly, a method for filling a medicated module that minimizes or eliminates the presence of air in the primary medicament cavity in the medicament cavity of the medicated module is provided.

According to an example, a medicated module is attachable to a drug delivery device that has a drug reservoir holding a first, primary medicament. The medicated module includes a slidable bung that comprises a bypass flow path and a medicament cavity. The medicament cavity is configured to store a second medicament. The medicated module also includes a bung housing for housing the slidable bung, and the bung housing comprises an inlet and an outlet. The slidable bung is configured to slide through the housing from a priming state to a dispensing state. In the priming state, the bypass flow path is in fluid communication with the inlet and outlet. Further, in the dispensing state, the medicament cavity is in fluid communication with the inlet and the outlet.

A particular benefit of Applicants' method and system is that the potential for air to be contained in the medicament that is ultimately dispensed from the medicated module is reduced or minimized. In an example, only a subset volume of the medicament volume filled into the medicated module is actually dispensed, and this subset volume is substantially free of air due to the configuration of the medicated module medicament cavity and the filling process. Further, another benefit of Applicants' method and system is that the system enables two discrete flow paths (i.e., a bypass flow path and a medicament flow path) to be sequentially engaged without axial displacement of medicated module components in line with a main axis of the delivery system.

A medicated module in accordance with Applicants' proposed concept can be designed for use with a drug delivery device with an appropriate compatible interface. However, it may be desirable to design the module in such a way as to limit its use to one exclusive primary drug delivery device (or family of devices) through employment of dedicated or coded features to prevent attachment of a non-appropriate medicated module to a non-matching device. In some situations it may be beneficial to ensure that the medicated module is exclusive to one drug delivery device while also permitting the attachment of a standard drug dispense interface to the device. This would allow the user to deliver a combined therapy when the module is attached, but would also allow delivery of the primary compound independently through a standard drug dispense interface in situations, such as, but not limited to, dose splitting or top-up of the primary compound.

In an example, the primary drug delivery device is used more than once and therefore is a multi-use device; however, the drug delivery device may also be a single use disposable device. Such a device may or may not have a replaceable reservoir of the primary drug compound, but Applicants' proposed concept is equally applicable to both scenarios. It is also possible to have a suite of different medicated modules for various conditions that could be prescribed as one-off extra medication to patients already using a standard drug delivery device. Should the patient attempt to reuse a previously used medicated module, features may be present that prevent reattachment to a primary drug delivery device or that prevent or discourage subsequent dosing through the needle via alternative means. For example, this medicated module may include a locking needle guard that is activated after a user delivers a dose from the medicated module. Other means of alerting the user may include some (or all) of the following:

Physical prevention of medicated module re-attachment to the primary drug delivery device once the module has been used and removed.

Physical / hydraulic prevention of subsequent liquid flow through the drug dispense interface once it has been used.

Physical locking of the dose setter and/or dose button of the primary drug delivery device.

Visual warnings (e.g., change in color and/or warning text/indicia within an indication window on the module once insertion and/or fluid flow has occurred).

Tactile feedback (presence or absence of tactile features on the outer surface of the module hub following use).

A further beneficial feature is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a perspective view of one possible drug delivery device that can be used with Applicants' proposed medicated module;
Figure 2a illustrates an example medicated module in accordance with Applicants' proposed concept;
Figure 2b illustrates a perspective, cross-sectional view of the example medicated module of Figure 2a;
Figure 3 illustrates an example stage of a filling and assembly process for the example medicated module of Figure 2a;
Figure 4 illustrates another example stage of a filling and assembly process for the example medicated module of Figure 2a;
Figure 5 illustrates another example stage of a filling and assembly process for the example medicated module of Figure 2a;
Figure 6 illustrates an example bypass function of the example medicated module of Figure 2a;
Figure 7 illustrates an example dispense function of the example medicated module of Figure 2a;
Figure 8 illustrates an example stage of a filling and assembly process for an example medicated module in accordance with Applicants' proposed concept;
Figure 9 illustrates another example stage of a filling and assembly process for the example medicated module of Figure 8;
Figure 10 illustrates an example bypass function of the example medicated module of Figure 8;
Figure 11 illustrates an example dispense function of the example medicated module of Figure 8;
Figure 12 illustrates another example medicated module in accordance with Applicants' proposed concept;
Figure 13 illustrates an example stage of a filling and assembly process for the example medicated module of Figure 12;
Figure 14 illustrates another example stage of a filling and assembly process for the example medicated module of Figure 12;
Figure 15 illustrates an example bypass function of the example medicated module of Figure 12;
Figure 16 illustrates an example dispense function of the example medicated module of Figure 16;
Figure 17 illustrates an example stage of a filling and assembly process for another example medicated module in accordance with Applicants' proposed concept;
Figure 18 illustrates another example stage of a filling and assembly process for the example medicated module of Figure 17;
Figure 19 illustrates an example bypass function of the example medicated module of Figure 17;
Figure 20 illustrates an example dispense function of the example medicated module of Figure 17;
Figure 21 illustrates an alternative slidable bung in accordance with an example of Applicants' proposed concept; and
Figure 22 illustrates another alternative slidable bung in accordance with an example of Applicants' proposed concept.

### DETAILED DESCRIPTION

Applicants' proposed concept administers a fixed predetermined dose of a secondary medicament and a variable dose of a primary or first medicament through a single output or drug dispense interface. Setting and dispensing the dose of the primary medicament by the user automatically determines the fixed dose of the second medicament. The proposed concept relates specifically to a method and system that involves (i) filling a medicated module with a larger volume of the second medicament than is required for dispense and (ii) capturing a known subset volume from this medicated module in a manner that minimizes the potential for air to be contained in the second medicament that is ultimately dispensed from the medicated module.

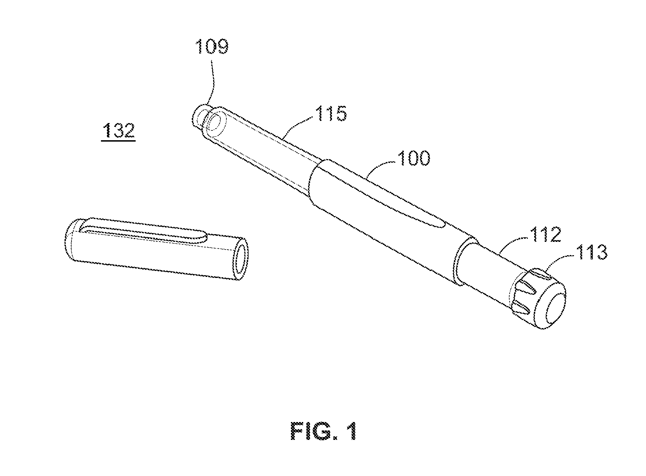

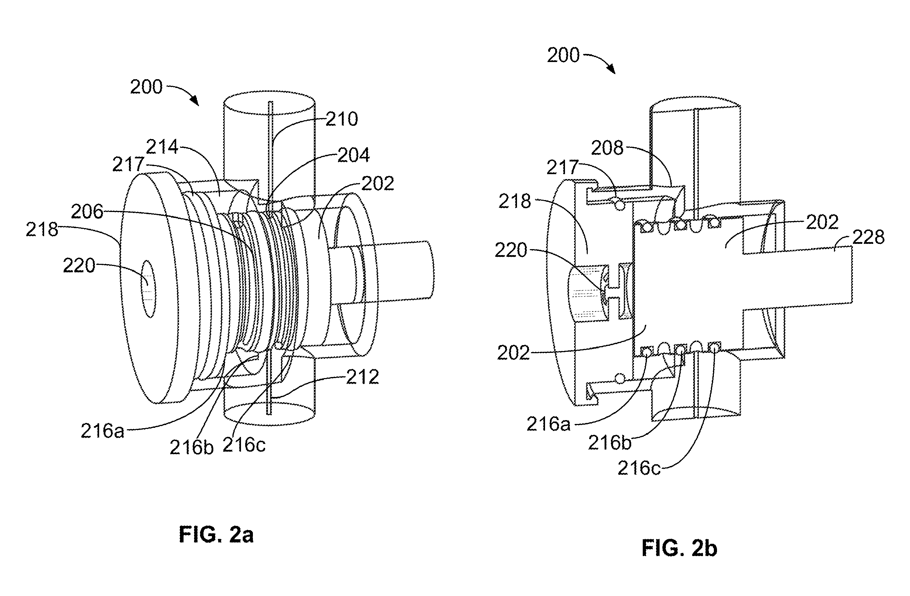

A medicated module in accordance with embodiments of Applicants' proposed concepts may be attached to a primary drug delivery device. Figure 1 illustrates one example drug delivery device 100 to which a medicated module, such as the example medicated modules depicted in Figures 2-20, can be attached. Specifically, a medicated module can be attached to the connection means 109 of distal end 132. A medicated module in accordance with Applicants' proposed concept is preferably self-contained and provided as a sealed and sterile disposable module that has an attachment means compatible to the attachment means 109 at the distal end 132 of device 100. Although not shown, the medicated module could be supplied by a manufacturer contained in a protective and sterile container, where the user would peel or rip open a seal or the container itself to gain access to the sterile medicated module. Further, the drug delivery device 100 includes a housing including a single dose setter 112. The dose setter 112 may be operably connected to a primary reservoir of medicament that may be stored in the drug delivery device 100, such as in cartridge holder 115. The user may use a dose dial button 113 in order to dial a user selectable dose of the primary medicament.

In an example, proper filling of a cavity of a medicated module may rely on accurate filling of the medicament and the incorporation of an amount of additional 'head-space' in the medicament cavity in order to accommodate manufacturing and assembly tolerances. Such a filling process may result in the presence of air in the medicament cavity after filling and closure which-in extreme tolerance conditions, for example-may have the potential to detrimentally reduce the delivered dose volume from the medicated module. One possible solution would be to tighten the tolerances of both the reservoir geometry and the tolerances on the fill accuracy of the filling equipment. However, this solution route would likely increase manufacturing costs and/or manufacturing complexity, and thus is not ideal for high volume, rapid throughput manufacturing.

Thus, in accordance with Applicants' proposed concept, a medicated module is provided that address these concerns. As mentioned above, the medicated module in accordance with Applicants' disclosure beneficially results in little or no air being present in the medicament cavity that stores the medicament that is ultimately dispensed. In particular, the proposed medicated module increases the nominal fill volume relative to the medicament cavity volume. Beneficially, this may reduce the challenges associated with filling the device due to the small volume (e.g., in the range of 20-60 microliters) required by the medicament cavity. In addition, the geometry of the medicated module reservoir region is such that, during the filling process, air is likely to accumulate in a known, controlled location (e.g., the top of the reservoir region). By capturing the excess air into a known, controlled location, it is then possible to design the medicated module to take out a known subset volume of this medicament from a region that is separate from this air-pocket, thereby reducing or minimizing the potential for air entrapment in the medicament cavity and thus minimizing its potential effect on the system dispense volume.

In general, a medicated module in accordance with Applicants' proposed concept includes a slidable bung that comprises a bypass flow path and a medicament cavity. The medicament cavity stores a second medicament. The medicated module also includes a bung housing for housing the slidable bung, and the bung housing comprises an inlet and an outlet. The slidable bung is configured to slide through the housing from a priming state to a dispensing state. In the priming state, the bypass flow path is in fluid communication with the inlet and outlet. Further, in the dispensing state, the medicament cavity is in fluid communication with the inlet and the outlet.

The medicated module may be configured and filled such that the medicated module is filled with a volume of medicament that is greater than the volume of the medicament that is ultimately dispensed from the system. At a given point before dispense, the medicament that is ultimately dispensed from the medicated module may be isolated from the excess medicament such that a known volume of medicament having minimal air present in the medicament is isolated. A known and substantial portion of the volume of the second medicament will be dispensed when the minimum required dose of the first medicament is dispensed from the primary device. In an example, the minimum required dose is 40 microliters or more; however, in other examples the minimum required dose may be less. So as to not waste medicament, which in some cases may be expensive, preferably at least 80% of the volume of the second medicament in the medicated module is dispensed when the minimum volume of the first medicament is dispensed from the primary drug delivery device 100.

In an embodiment, the slidable bung is orthogonal to a main axis of the medicated module and, when activated, slides to engage the medicament cavity with the flow path. Thus, no axial movement of the slidable bung is required to alternate between a prime state and a dispense state.

A first example medicated module 200 is described with reference to Figures 2a-7. Figure 2a shows a perspective view of the medicated module 200, and Figure 2b shows a perspective, cross-sectional view of the medicated module 200. For clarity, example internal features of the medicated module 200 are depicted in Figure 2a. Medicated module 200 includes a slidable bung 202, which includes a bypass flow path 204 and a medicament cavity 206. The medicated module 200 also includes a bung housing 208 for housing the slidable bung 202. A portion of the bung housing 208 may be a tubular portion that generally corresponds to the shape of the slidable bung 202. In the example shown, the bung housing 208 comprises an oval-shaped tubular element, and this oval shape helps to rotationally constrain the slidable bung 202. Other slidable-bung shapes are possible as well.

The bung housing 208 also includes an inlet channel 210 and an outlet channel 212. Respective needle cannula may be inserted into the inlet 210 and the outlet 212. For example, respective needle cannula may be insert molded or bonded into the inlet channel 210 and the outlet channel 212. The needle cannula in the inlet channel 210 may establish fluid communication with a drug reservoir of the primary drug delivery device to which the medicated module 200 is attached. Further, the needle cannula in the outlet channel 212 may serve as an output needle for piercing and dispensing medicament into an injection site.

Although not shown, medicated module 200 may include an outer body that houses some or all of the components depicted in Figure 2a. Further, the medicated module may include a needle guard that acts as a needle guard for the output needle (not shown) that is held in the outlet 212. Such a needle guard may be similar to needle guards now know in the art or later developed.

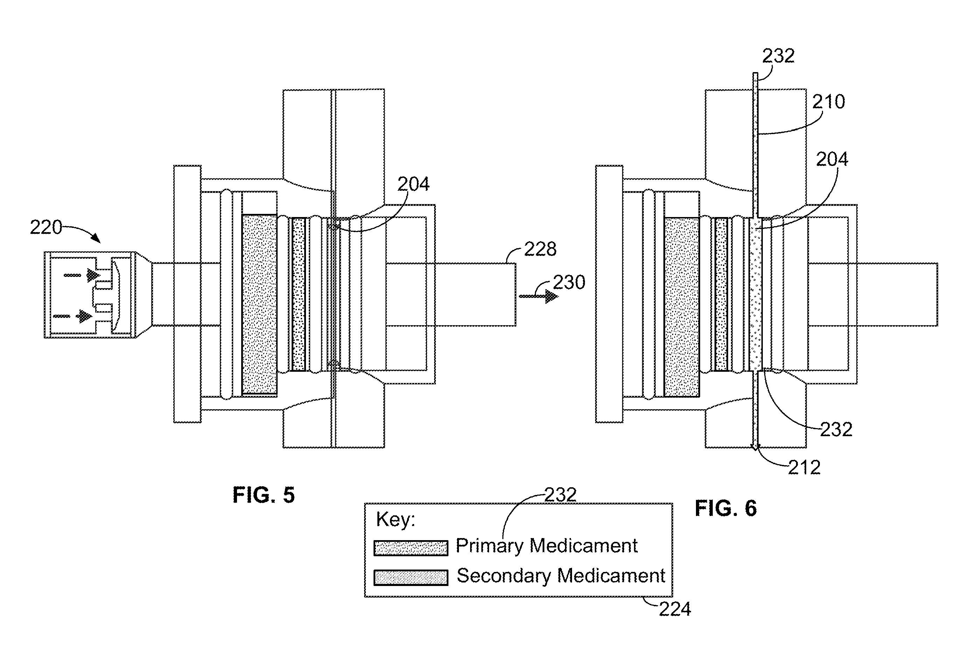

The slidable bung 202 is configured to slide through the housing 208 from a priming state to a dispensing state. In this example, the slidable bung 202 is configured to slide laterally through the housing in a horizontal direction. However, note that the bung may slide through the housing in other directions, such as at an angle. In the priming state, the bypass flow path 204 is in fluid communication with both the inlet channel 210 and outlet channel 212. Thus, a user may prime the drug delivery system (i.e., the drug delivery device 100 and attached medicated module 200) with the first medicament 232 (see Figure 6) stored in the drug delivery device 100. Although Figure 6 depicts only the medicated module 200, it should be appreciated that the medicated module may be attached to the drug delivery device 100, and that the first, primary medicament 232 is stored in drug delivery device 100. In the dispensing state, the medicament cavity 206 is in fluid communication with the inlet 210 and the outlet 212.

The bung housing 208 includes a medicament-reservoir portion 214. The medicated module 200 may also include at least one sealing element, such as seals 216a-c. The seals may be configured to seal at least one of the bypass flow path 204 and the medicament cavity 206. The seals for the slidable bung may be manufactured from a thermoplastic elastomer (TPE), silicon, or some other compliant, inert material, with the main component 202 being manufactured from cyclo-olefin polymer (COP), or another similarly inert moldable material. The construction may be achieved via a co-molding process, or alternatively the seals may be provided by separate components (e.g., o-rings) assembled onto the main slidable bung component. In this example, the seals 216a-c are o-ring seals; however, other sealing elements are possible as well.

The medicated module 200 may also include a reservoir cap 218 that comprises a valve 220. The cap 218 and valve 220 operate to seal the medicament in the medicated module 200 and allow for pressure equalization when the medicated module 200 is actuated to isolate in the medicament cavity 206 the second medicament to be dispensed.

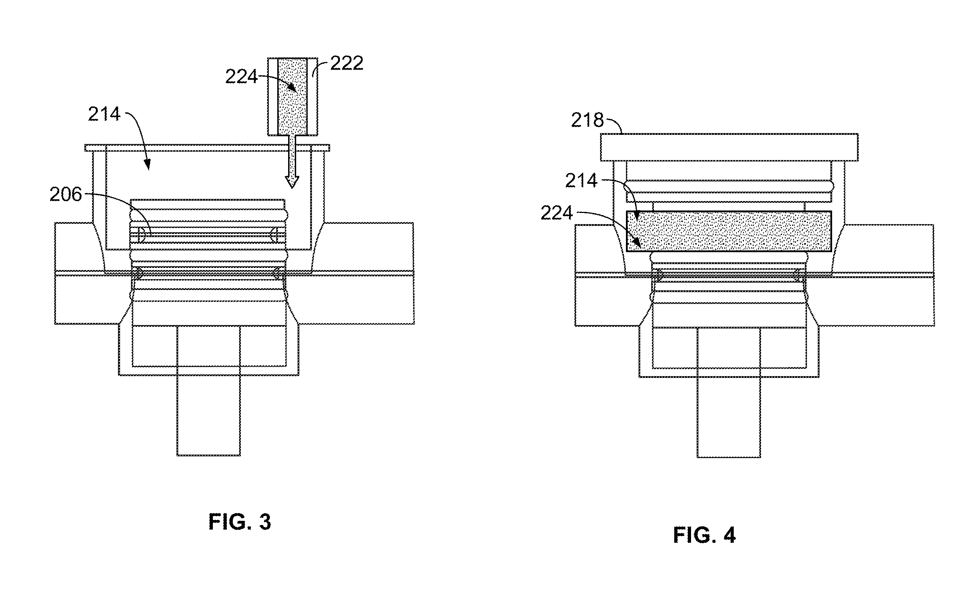

As mentioned above, beneficially, a medicated module comprising the parts illustrated in Figures 2a-b may be filled and actuated so that there is little or no air present in the medicament cavity from which the second medicament is ultimately dispensed. In particular, the medicated module in accordance with Applicants' proposed concept may isolate a known volume in the medicament cavity such that air trapped in the isolated volume is minimized, thereby minimizing dispense-accuracy variability caused by expulsion of air from the storage container. Accordingly, a known, controlled amount of medicament may be dispensed from the medicated module. An example assembly and filling process for medicated module 200 is described in detail below with respect to Figures 3-5.

In this example, the medicated module 200 is actuated during the assembly process. That is, the second medicament which is dispensed from the medicated module is isolated in the medicament cavity during the assembly process. More medicament than will ultimately be dispensed is filled into the medicated module (and in particular, into the medicament reservoir 214 of the outer housing 208). When actuated during the assembly and filling process, the medicated module 200 isolates in the medicament cavity 206 the medicament to be dispensed.

With reference to Figure 3, the medicated module 200 may be oriented such that the reservoir 214 faces upwards. A filling assembly 222 may then fill the secondary medicament 224 into the medicament reservoir 214. As seen in Figure 3, the medicament reservoir 214 surrounds the medicament cavity 206. In particular, the volume of the medicament reservoir 214 is greater than the volume of the medicament cavity 206. The medicament 224 may be filled to a fill level such that the medicament cavity 206 is completely filled with medicament 224. Thus, prior to actuation of the slidable bung, the medicament-reservoir portion is in fluid communication with the medicament cavity, wherein the medicament-reservoir portion is at least partially filled with the second medicament such that the medicament cavity is completely filled with the second medicament.

When the medicament reservoir 214 is filled to a sufficient fill level, the reservoir cap 218 may be secured to the bung housing 208, as shown in Figure 4. Air trapped in the medicament reservoir 214 is likely to float free from the medicament cavity 206. Thus, the potential for air to be trapped in the medicament cavity 206 is minimized.

In an example, the reservoir cap 218 may comprises a snap feature and may snap into place. However, other connection mechanisms are possible. In addition, the cap may include a seal 217, such as an o-ring, that seals the medicament 224.

When the cap 218 is sealed, the medicated module 200 may be actuated to isolate in the medicament cavity 206 the medicament 224. Figure 5 shows the medicated module 200 after the module 200 is actuated during the assembly process. In this example, the assembly equipment may actuate the slidable bung 202 by moving the bung actuator 228 in lateral direction 230. This actuation isolates and seals the medicament 224 in the medicament cavity 206. As mentioned above, minimal or no air is present in the medicament 224 that is isolated in medicament cavity 206.

During the actuation, pressure in the reservoir 214 is allowed to equalize through valve 220. In particular, as depicted in Figure 5, air passes through the valve 220 to equalize pressure in the reservoir 214. Movement of the bung 202 during the actuation establishes fluid communication between the bypass flow path 204, inlet 210, and outlet 212. Thus, the medicated module 200 is in a priming state after the actuation.

Prior to delivering a dose, a user may prime the drug delivery device 100 and medicated module 200. In particular, the user may prime the drug delivery device 100 and medicated module 200 with the first medicament 232 by forcing a priming dose of the first medicament 232 through the bypass flow path 204. Figure 6 depicts the flow of the primary medicament 232 through the bypass flow path 204. In particular, the first medicament 232 may flow from the drug delivery device 100 to the inlet 210, through the bypass flow path 204, and then out the outlet 212. As seen in Figure 6, the bypass path 204 avoids contact with the second medicament 224 stored in the medicament cavity 206. Thus, none of the second medicament 224 is used in priming the device. Note that although the example medicated module 200 includes a bypass path to facilitate this priming capability, other medicated modules in accordance with Applicants' proposed concept may not include such a bypass function.

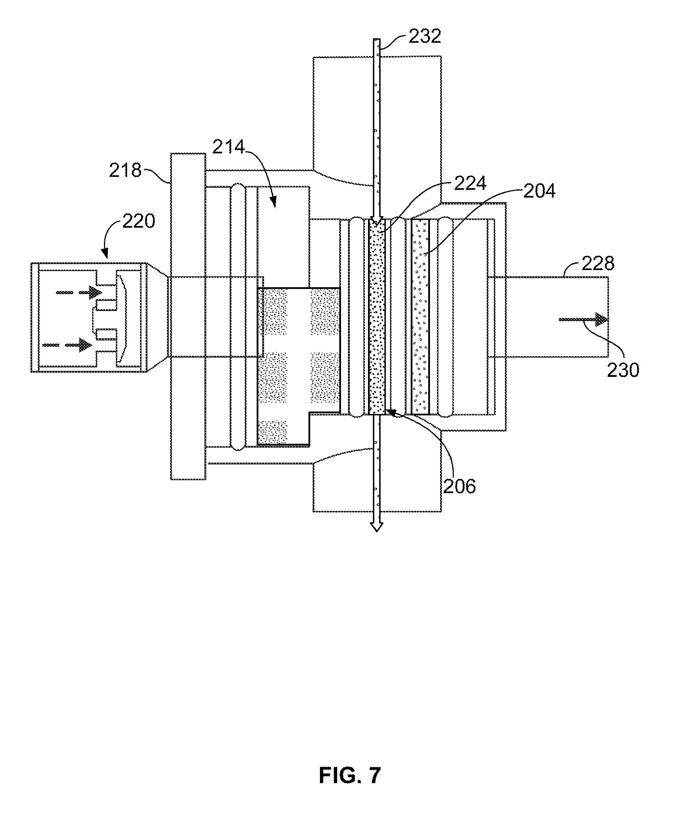

After priming of the drug delivery device 100, the medicated module 200 may be switched from the priming state to a dispensing state. This switch between the two states is described in detail with reference to Figure 7. To switch from the priming state to the dispensing state, the slidable bung 202 may be further actuated in the lateral direction 230. This further actuation of the slidable bung 202 could be accomplished in numerous ways. For example, this further actuation could be automatically caused by the dispense process. For instance, this further actuation could be integrated into the movement of a needle guard (not shown) of the medicated module. In particular, the needle guard may be configured to automatically actuate the slidable bung as the needle guard to be moved in a proximal direction. In another example, the further actuation may involve the release of a stored energy (such as a sprung element) that may cause the desired lateral movement. Additionally or alternatively, this further actuation could be caused by a separate user action during or prior to the dispense process. For example, a user may be required to push or pull the bung actuator 228 in order to force the further actuation of the bung.

Note that if the medicated module 200 is configured to be actuated further by the user directly, this action may also unlock a single-use needle guard mechanism that may be present on the medicated module. This arrangement may ensure that a user only injects a dose with the secondary medicament fluidically engaged to the outlet 212. In addition, such an arrangement may also be configured to prevent re-use of an already used medicated module.

During this further actuation to move to the dispense state, pressure in the medicament reservoir 214 is once again allowed to equalize through valve 220. However, in other examples, a different means may be used to equalize the pressure during the actuation phases of the medicated module 200. For instance, the pressure in the medicament reservoir 214 could be managed by filling under elevated pressure or providing a flexible membrane which deforms to equalize the pressure. Other pressure-equalization methods are possible as well.

After the medicated module 200 is switched to the dispensing state, a combination of the first medicament 232 and the second medicament 224 may then be dispensed from the drug delivery device 100 and medicated module 200. The dose of the first medicament 232 from the drug delivery device 100 may be set in a usual manner (e.g., by dialing out an appropriate number of units of the primary medicament 232 with a dose dial). Dispense of the second medicament 224 and the first medicament 232 may then be achieved via activation of the dosing mechanism of the drug delivery device 100. As shown in Figure 7, the medicament 232 may flow in the distal direction from the drug delivery device 100 to the medicated module 200, and this flow of first medicament 232 may force the second medicament 224 from the medicament cavity 206.

After the user finishes dispensing the first medicament 232 and the second medicament 224, the user may remove the output needle from the injection site. Then, the depleted medicated module 200 may be disconnected from the drug delivery device 100 and disposed of. Assuming that the drug delivery device 100 still holds some first medicament 232, the drug delivery device 100 may be reused by the user/patient as required.

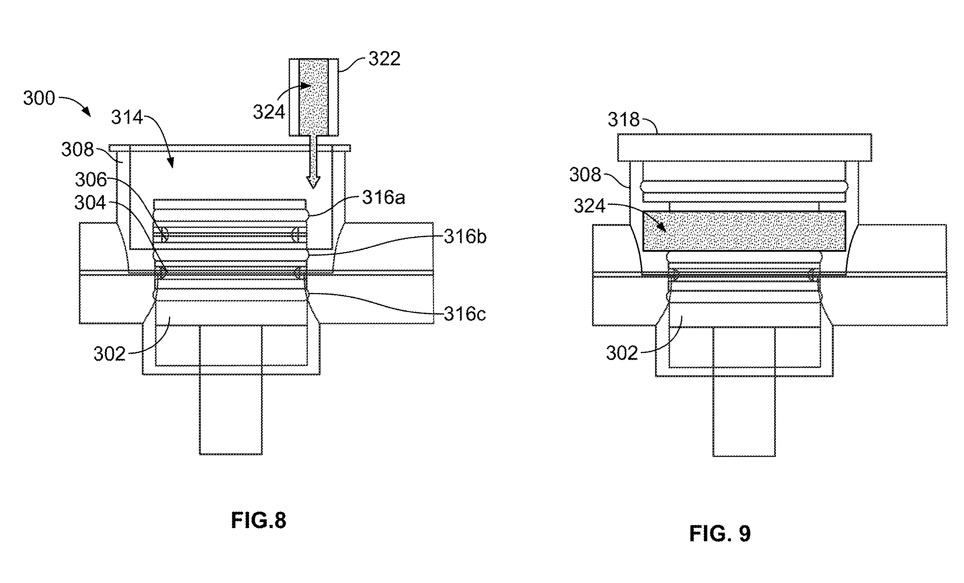

A second example medicated module 300 is described with reference to Figures 8-11. This example is similar in many respects to the example of Figures 2a-7 and thus is not described in as great detail. Note that many of the possibilities and permutations described above with respect to medicated module 200 are also possible with respect to medicated module 300. However, rather than the initial actuation to isolate the second medicament in the medicament cavity occurring during the assembly process, the medicated module 300 is configured so that the user actuates the medicated module 300 to isolate the second medicament in the medicament cavity.

Figures 8-9 depict an example filling process for medicated module 300. The medicated module 300 may be oriented such that the reservoir 314 faces upwards. A filling assembly 322 may then fill the secondary medicament 324 into the medicament reservoir 314. The volume of the medicament reservoir 314 is greater than the volume of the medicament cavity 306. The medicament 324 may be filled to a fill level such that the medicament cavity 306 is completely filled with medicament. When the medicament reservoir 314 is filled to a sufficient fill level, the reservoir cap 318 may be secured to the bung housing 308, as shown in Figure 9. Air trapped in the medicament reservoir 314 is likely to float free from the medicament cavity 306.

When medicated module 300 is attached to a drug delivery device, such as drug delivery device 100, the bypass flow path 304 may be engaged with the first, primary medicament 332 in the primary drug delivery device 100.

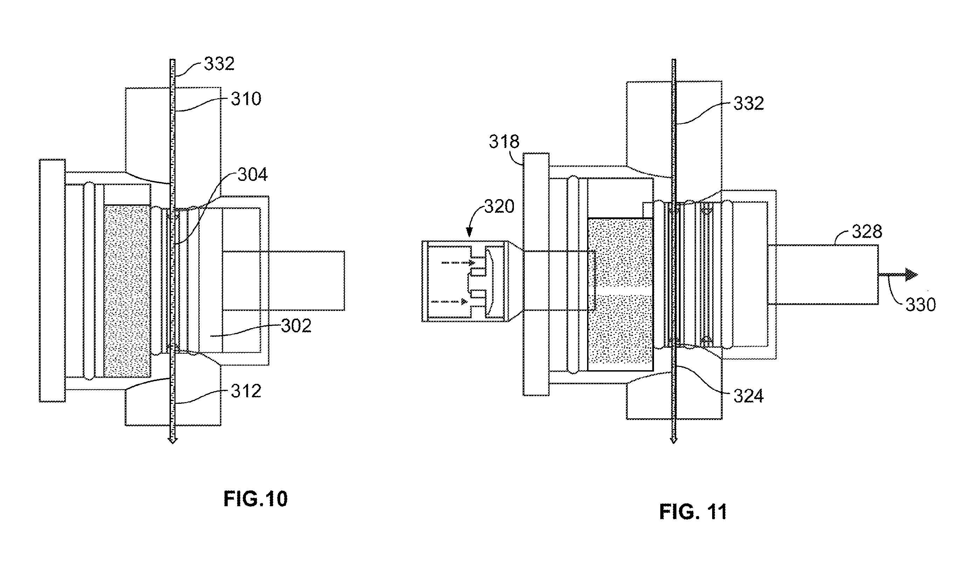

Prior to delivering a dose, a user may prime the drug delivery device 100 and medicated module 300. In particular, the user may prime the drug delivery device 100 and medicated module 300 with the first medicament 332 stored in the drug delivery device 100 by forcing a priming dose of the first medicament 332 through the bypass flow path 304. Figure 10 depicts the flow of the medicament 332 through the bypass flow path 304 of the medicated module 300. In particular, the first medicament 332 may flow from the drug delivery device 100 to the inlet 310, through the bypass flow path 304, and then out the outlet 312.

After priming, the medicated module 300 may be switched from the priming state to a dispensing state. This switch between the two states is described in detail with reference to Figure 11. The slidable bung 302 may be actuated in the lateral direction 330. Similar to the further actuation of the slidable bung 302, this initial actuation of the slidable bung 302 could be accomplished in numerous ways. For example, this actuation could be automatically caused by the dispense process. For instance, this further actuation could be integrated into the movement of a needle guard (not shown) of the medicated module. The needle guard may be configured to automatically actuate the slidable bung as the needle guard to be moved in a proximal direction. Additionally or alternatively, this actuation could be caused by a separate user action during or prior to the dispense process. For example, a user may be required to push or pull the bung actuator 328 in order to force the further actuation of the slidable bung 302. During this actuation, pressure in the medicament reservoir 314 is allowed to equalize through valve 320. However, in other examples, a different means may be used to equalize the pressure during the actuation phases of the medicated module 300.

After the medicated module 300 is switched to the dispensing state, a combination of the first medicament 332 and the second medicament 324 may be dispensed from the drug delivery device and medicated module.

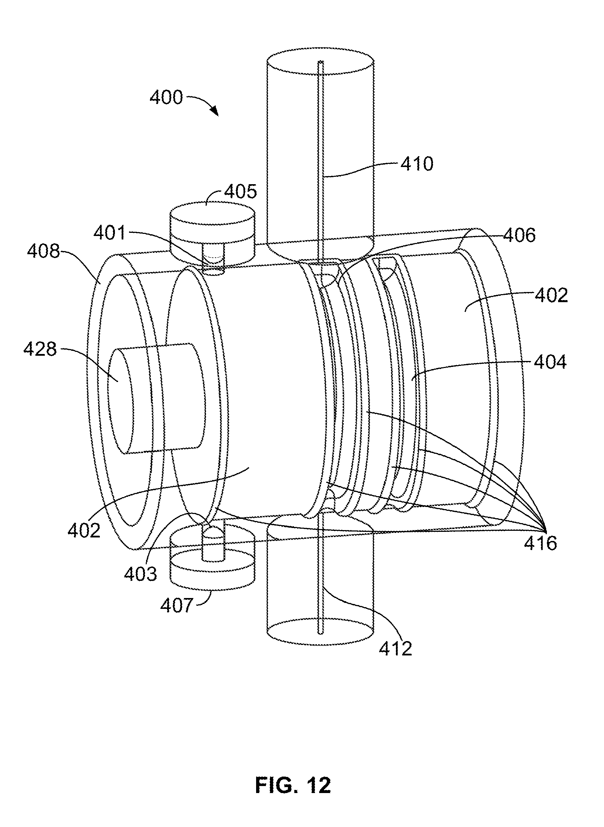

A third example medicated module 400 is described with reference to Figures 12-16. This example is similar in many respects to the example medicated modules discussed above and thus is not described in as great detail. Note that many of the possibilities and permutations described above with respect to the medicated modules above are possible with respect to medicated module 400. However, rather than the second medicament being filled into a medicament reservoir that surrounds a medicament cavity, the medicament may be filled into a medicament cavity via a fill channel.

In particular, the medicated module 400 includes a fill channel 401 that is used to fill the medicament cavity 406. Medicated module 400 also includes a vent channel 403. Further, similar to the examples discussed above, medicated module 400 includes a slidable bung 402 having a bypass flow path 404, a medicament cavity 406, and seals 416. The module 400 also includes a bung housing 408 that includes an inlet channel 410 and an outlet channel 412. In this example, the seals 416 are localized sealing beads. In an example, the localized sealing bead 416 may be twin-shot molded into the slidable bung 402. The sealing beads may be composed of, for example, thermoplastic elastomers (TPE) or silicon; however, other sealing materials are possible as well.

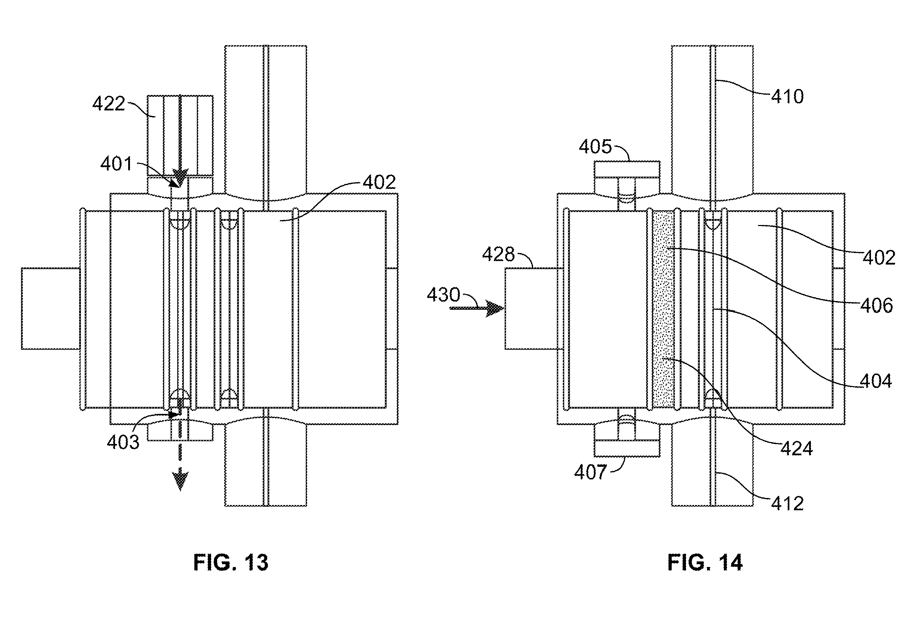

Figure 13-14 depict example steps for an example filling process for medicated module 400. A filling apparatus 422 may be brought up against the filling channel 401, and the second medicament 424 may be filled into the medicament cavity 406. As seen in Figure 13, air may be forced through the medicament cavity 406 and then escape through the vent channel 403. When the second medicament 424 fills the medicament cavity 406, a fill cap 405 and vent cap 407 may be secured over the fill channel 401 and vent channel 403, respectively, to seal the channels. In an example, the channels may contain a residual fill medicament.

Further, as seen in Figure 14, the slidable bung 402 may be actuated by the assembly equipment to isolate the medicament in the medicament cavity 406. In an example, the assembly equipment may push the bung actuator 428 in lateral direction 430. This actuation isolates and seals the medicament 424 in the medicament cavity 406. In addition, the actuation aligns the inlet channel 410 and outlet channel 412 with the bypass flow path 404.

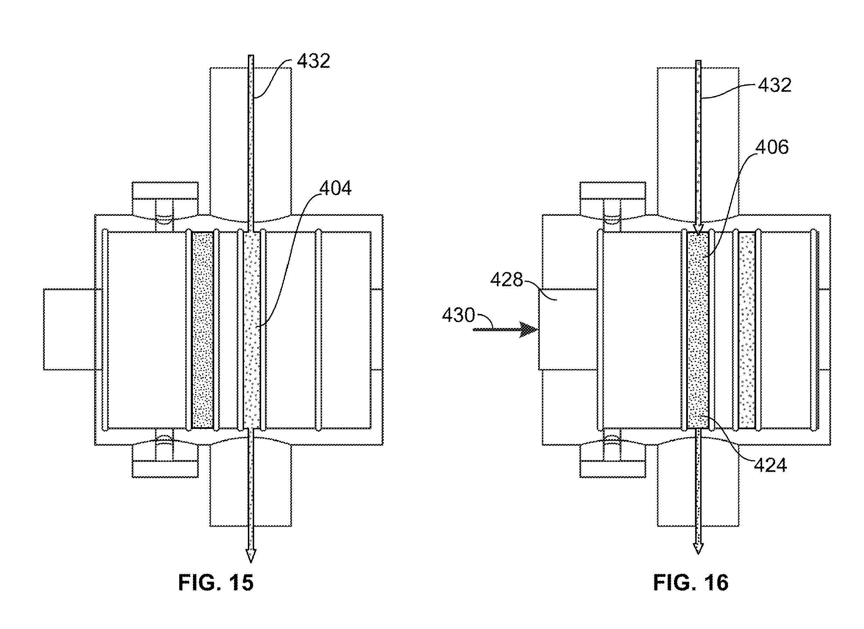

Figure 15 demonstrates the bypass function of medicated module 400. Similar to the example of Figures 2a-7, a user may prime the system with the first medicament from the drug delivery device 100. The medicated module 400 may then be switched from a priming state to a dispense state. This may be accomplished in the ways such as those discussed above. In the dispense state, the inlet 410 and outlet 412 are aligned with the medicament cavity 406. Thus, both medicaments 424, 432 may then be dispensed, as shown in Figure 16.

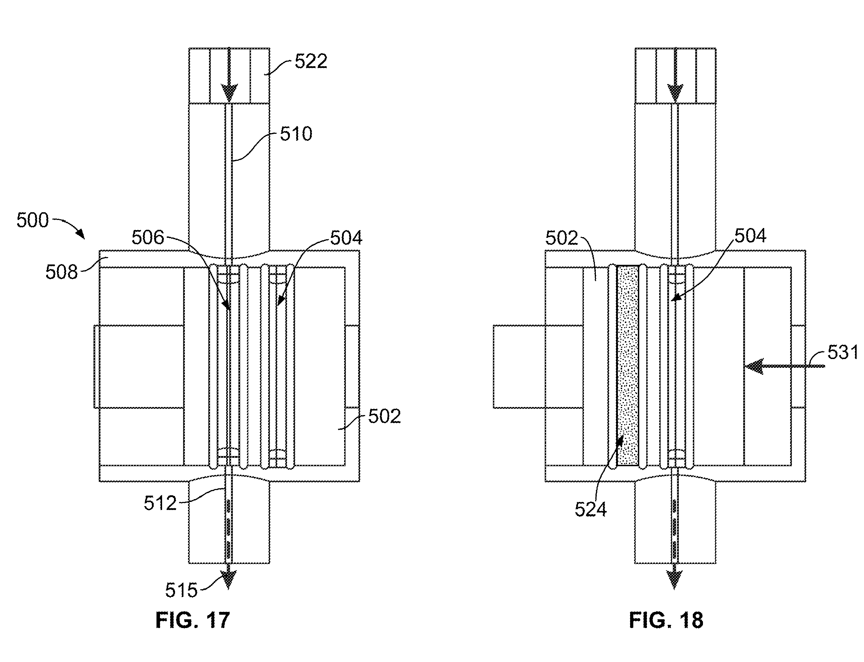

A fourth example medicated module 500 is described with reference to Figures 17-20. This example is similar in many respects to the example medicated modules discussed above and thus is not described in as great detail. Once again, note that many of the possibilities and permutations described above with respect to the medicated modules above are also possible with respect to medicated module 500. However, with comparison to medicated module 400, rather than having a dedicated filling channel and vent channel for filling the medicament cavity, the filling apparatus 522 connects directly to the inlet channel 510. In this example, the outlet channel 512 acts as a vent during filling of the medicament cavity 506 with the second medicament 524.

Medicated module 500 includes a slidable bung 502 and a bung housing 508. With reference to Figure 17, at an initial stage of the filling process, the medicament cavity 506 is aligned with the inlet channel 510 and the outlet channel 512. Medicament 524 is then dispensed into the medicament cavity 506 with the filling apparatus 522. During this process, air that was held in the cavity 506 escapes via the outlet channel 512, as indicated by arrow 515. After the air is purged and the medicament cavity 506 is completely filled, the bung 502 may be actuated (e.g., forced in direction 531) to isolate and seal the medicament cavity 506, as shown in Figure 18. After this occurs, the filling nozzle 522 may force air through the inlet 510 to drive any residual medicament from the medicated module 500, in particular from the inlet channel 510 and outlet channel 512.

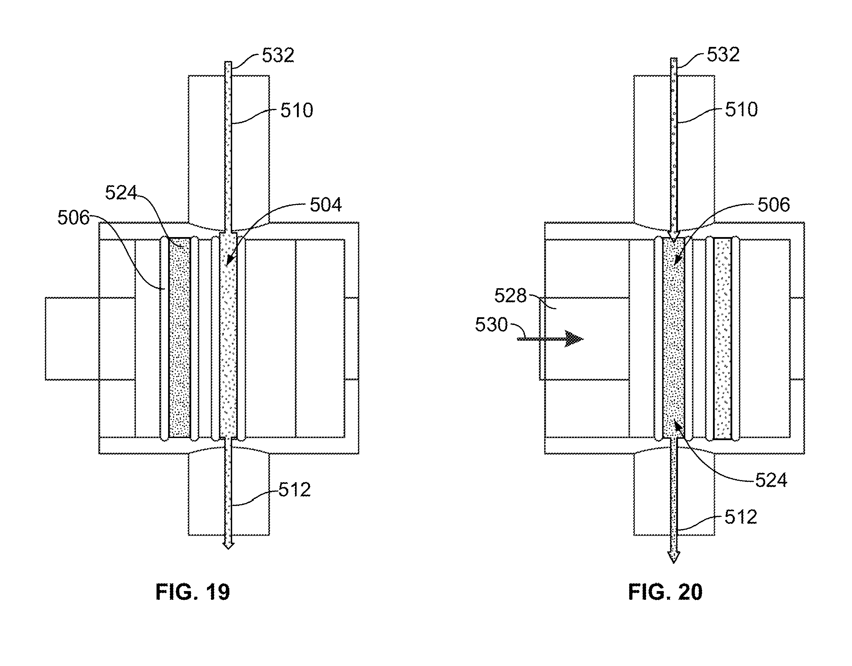

Figure 18 demonstrates the bypass function of medicated module 500. Similar to the examples above, a user may prime the system with the first medicament from the drug delivery device 100. In particular, primary medicament 532 from the drug delivery device 100 may be used to prime the device. The medicated module 500 may then be switched from a priming state to a dispense state. In particular, bung actuator 528 may be forced in lateral direction 530 to facilitate the switch to the dispense state. This may be accomplished in the ways such as those discussed above. In the dispense state, the inlet 510 and outlet 512 are aligned with the medicament cavity 506. Thus, both medicaments 524, 532 may then be dispensed, as shown in Figure 20.

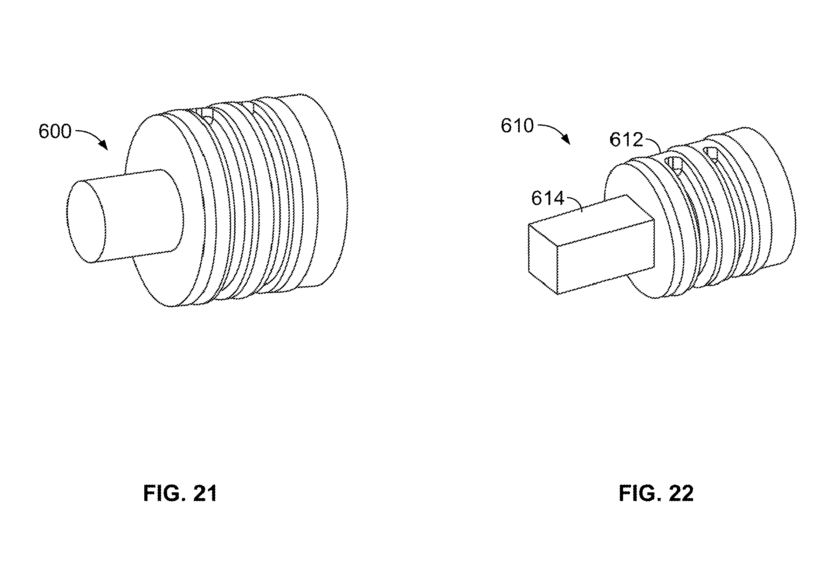

The slidable bung in accordance with Applicants' proposed concept may be manufactured in a variety of ways. For example, the slidable bung may be manufactured as a one-piece compliant bung. As an alternative to the one-piece compliant bung, for drug stability reasons, it may be desirable to mold the body from a rigid inert polymer (e.g., an inert polymer such as cyclo-olefin polymer (COP), high-density polyethylene (HDPE), or polypropylene (PP)) and to create the sealing interface using o-rings, as depicted in the slidable bung 600 shown in Figure 21. A second alternative would be to twin-shot the compliant material over the inert polymer body.

In the medicated module examples described above, the slidable bung is constrained rotationally relative to the bung housing by utilizing an ovalised section. However, an ovalised section may not be required to rotationally constrain the slidable bung. For instance, an alternative would be to use a slidable bung having a circular section with a non-circular bung actuator such that the bung actuator constrains the bung rotationally. For example, Figure 22 depicts a slidable bung 610 that has a circular section 612 and a non-circular bung actuator 614.

As described above, Applicants' proposed concept beneficially allows for a medicated module that minimizes the potential for air to be contained in the medicament that is ultimately dispensed from the system. Thus, such a medicated module may administer a highly accurate dose of the second medicament. The highly accurate amount of medicament dispensed from the medicated module may be varied by altering the shape and/or size of the medicament cavity.

The connection or attachment between the medicated module of the above described examples may contain additional features (not shown), such as connectors, stops, splines, ribs, grooves, and the like design features, that ensure that specific medicated module are attachable only to matching drug delivery devices. Such additional features would prevent the insertion of a non-appropriate medicated module to a non-matching injection device.

The shape of the medicated module may generally be a cylindrical body or any other geometric shape suitable for defining a fluid reservoir or for containing discrete self-contained reservoir of the medicament in the medicated module and for attaching one or more needle cannula. Generally, the medicated module can be manufactured from glass or other drug contact suitable material. The integrated output needle can be any needle cannula suitable for subcutaneous or intramuscular injection. In an example, the medicated module is provided by a drug manufacturer as a stand-alone and separate device that is sealed to preserve sterility. The sterile seal of the module is preferably designed to be opened automatically, e.g. by cutting, tearing or peeling, when the medicated module is advanced or attached to the drug delivery device by the user.

The medicated module of Applicants' concept may be designed to operate in conjunction with a multiple use injection device, preferably a pen-type multi-dose injection device, similar to what is illustrated in Figure 1. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose and preferably a multi-dose device, however, in some cases it may be beneficial to use a single dose, disposable device.

A typical drug delivery device contains a cartridge or other reservoir of medication. This cartridge is typically cylindrical in shape and is usually manufactured from glass. The cartridge is sealed at one end with a rubber bung and at the other end by a rubber septum. The drug delivery device is designed to deliver multiple injections. The delivery mechanism is typically powered by a manual action of the user, however, the injection mechanism may also be powered by other means such as a spring, compressed gas or electrical energy.

Exemplary embodiments of the present invention have been described. Those skilled in the art will understand, however, that changes and modifications may be made to these embodiments without departing from the true scope and spirit of the present invention, which is defined by the claims.

### List of references

- 100: drug delivery device
- 109: connection means
- 112: dose setter
- 113: dose dial button
- 115: cartridge holder
- 132: distal end of drug delivery device
- 200: medicated module
- 202: slidable bung
- 204: bypass flow path
- 206: medicament cavity
- 208: bung housing
- 210: inlet channel
- 212: outlet channel
- 214: medicament-reservoir portion
- 216a-c: seals
- 217: seal
- 218: reservoir cap
- 220: valve
- 222: filing assembly
- 224: secondary medicament
- 228: bung actuator
- 230: lateral direction
- 232: first medicament
- 300: medicated module
- 302: slidable bung
- 304: bypass flow path
- 306: medicament cavity
- 308: bung housing
- 310: inlet
- 312: outlet
- 314: medicament-reservoir portion
- 318: reservoir cap
- 320: valve
- 322: filling assembly
- 324: secondary medicament
- 328: bung actuator
- 330: lateral direction
- 332: primary medicament / first medicament
- 400: medicated module
- 401: fill channel
- 402: slidable bung
- 403: vent channel
- 404: bypass flow path
- 405: fill cap
- 406: medicament cavity
- 407: vent cap
- 408: bung housing
- 410: inlet channel
- 412: outlet channel
- 416: seals
- 422: filling apparatus
- 424: secondary medicament
- 428: bung actuator
- 430: lateral direction
- 432: primary medicament / first medicament
- 500: medicated module
- 502: bung
- 506: medicament cavity
- 508: bung housing
- 510: inlet channel
- 512: outlet channel
- 515: arrow
- 522: filling apparatus
- 524: second medicament
- 528: bung actuator
- 530: lateral direction
- 531: direction
- 532: primary medicament / first medicament
- 600: slidable bung
- 610: slidable bung
- 612: circular section
- 614: non-circular bung actuator

## Claims

1. A medicated module attachable to a drug delivery device, the drug delivery device having a drug reservoir holding a first medicament, the medicated module comprising:
a slidable bung (202, 302, 402, 502), wherein the slidable bung (202, 302, 402, 502) comprises a bypass flow path (204, 304, 404, 504) and a medicament cavity (206, 306, 406, 506), wherein the medicament cavity (206, 306, 406, 506) stores a second medicament (224, 324, 424, 524); and
a bung housing (208, 308, 408, 508) for housing the slidable bung (202, 302, 402, 502), wherein the bung housing comprises an inlet channel (210, 310, 410, 510) and an outlet channel (212, 312, 412, 512);
wherein the slidable bung (202, 302, 402, 502) is configured to slide through the housing from a priming state to a dispensing state, wherein, in the priming state, the bypass flow path (204, 304, 404, 504) is in fluid communication with the inlet channel (210, 310, 410, 510) and outlet channel (212, 312, 412, 512), and wherein, in the dispensing state, the medicament cavity (206, 306, 406, 506) is in fluid communication with the inlet channel (210, 310, 410, 510) and the outlet channel (212, 312, 412, 512).

2. The medicated module of claim 1, wherein (i) in the priming state the medicated module (200, 300, 400, 500) is configured to be primed with the first medicament (232, 332, 432, 532), and (ii) in the dispensing state the medicated module (200, 300, 400, 500) is configured to dispense the first medicament (232, 332, 432, 532) and the second medicament (224, 324, 424, 524).

3. The medicated module of claims 1 or 2 , wherein the slidable bung (202, 302, 402, 502) is configured to slide laterally through the bung housing (208, 308, 408, 508).

4. The medicated module of claims 1-3, further comprising at least one seal (216a-c, 416), wherein each of the at least one seal is configured to seal at least one of the bypass flow path (204, 404) and the medicament cavity (206, 406).

5. The medicated module of claim 4, wherein each of the at least one seal comprises an o-ring (216a-c).

6. The medicated module of any of the preceding claims, wherein the housing comprises a medicament-reservoir portion (214, 314), wherein the medicament-reservoir portion (214, 314) is capable of holding a volume greater than the medicament cavity (206, 306), and
wherein, prior to actuation of the slidable bung (202, 302), the medicament-reservoir portion (214, 314) is in fluid communication with the medicament cavity (206, 306), wherein the medicament-reservoir portion (214, 314) is at least partially filled with the second medicament (224, 324) such that the medicament cavity (206, 306) is completely filled with the second medicament (224, 324).

7. The medicated module of claim 6, further comprising a reservoir cap (218) having a valve (220) configured to equalize pressure in the medicament-reservoir portion (214) when the slidable bung (202) is actuated.

8. The medicated module of any of the preceding claims, further comprising:
a bung actuator (228, 328, 428, 528), wherein the bung actuator is movable in a lateral direction (230, 330, 430, 530) to actuate the slidable bung (202, 302, 402, 502).

9. The medicated module of claim 8, wherein the slidable bung (610) comprises a non-circular bung actuator (614), wherein the non-circular bung actuator rotationally constrains the slidable bung (610) to the outer housing.

10. The medicated module of claim 8, wherein the slidable bung (202, 302, 402, 502) is has a shape that prevents rotation of the bung.

11. The medicated module of any of the preceding claims wherein the second medicament (224, 424, 524) is isolated in the medicament cavity (206, 406, 506) upon a preliminary actuation of the slidable bung (202, 402, 502) during an assembly process.

12. The medicated module of any of the preceding claims, wherein the second medicament (324) is isolated in the medicament cavity (306) upon an actuation of the slidable bung (302) during an injection process.

13. The medicated module of any of the preceding claims wherein the second medicament (324) is isolated in the medicament cavity (306) upon an actuation of the slidable bung (302) resulting from a direct user action.

14. The medicated module (400) of claim 1, further comprising:
a filling channel (401);
a vent channel (403); and
wherein the medicament cavity (406) is configured to be filled through the filling channel (401), and wherein the vent channel (403) allows air to escape from medicament cavity (406) during a filling process.

15. The medicated module (400) of claim 14, further comprising:
a filling-channel cap (405), wherein the filling-channel cap (405) seals the filling channel (401); and
a vent-channel cap (407), wherein the vent-channel cap (407) seals the vent channel (403).

16. The medicated module of claim 1 wherein at least one of the inlet channel (510) is used for filing the medicament cavity (506) or the outlet channel (512) is used for venting the medicament cavity (506).
